# EUROPEAN PATENT SPECIFICATION

(11) **EP 2 229 206 B1**
(45) Date of publication and mention of the grant of the patent: **13.04.2016**
(21) Application number: 08866616.9
(22) Date of filing: 18.12.2008
(51) Int. Cl.: A61M 16/00, A61M 16/04, A61B 5/087, A61M 16/22, A61M 16/08

(54) **ENDOTRACHEAL TUBE FOR MEASURING LUNG FLOW AND METHOD FOR MANUFACTURING A MEDICAL SYSTEM COMPRISING SAID ENDOTRACHEAL TUBE**
ENDOTRACHEALTUBUS ZUR LUNGENFLUSSMESSUNG UND VERFAHREN ZUR HERSTELLUNG EINES DEN ENDOTRACHEALTUBUS AUFWEISENDEN MEDIZINISCHEN SYSTEMS
TUBE ENDOTRACHÉAL POUR MESURER UN ÉCOULEMENT PULMONAIRE ET PROCÉDÉ POUR FABRIQUER UN SYSTÈME MÉDICAL COMPRENANT LEDIT TUBE ENDOTRACHÉAL

(30) Priority: 26.12.2007 US 9174 P
(43) Date of publication of application: 22.09.2010
(73) Proprietor: Covidien LP, Mansfield, MA 02048 (US)
(72) Inventor: BLYAKHMAN, Yakov, Pleasanton CA 94566 (US); WANG, Hui, San Ramon CA 95035 (US); LI, Li, Milpitas CA 95035 (US); PETERSEN, James, Pleasanton CA 94588 (US); VANDINE, Joseph, Newark CA 94560 (US)
(74) Representative: Kinsler, Maureen Catherine
(86) International application number: PCT/US2008/087483
(87) International publication number: WO 2009/085984

(56) References cited:
- EP-A- 0 850 652
- WO-A-01/23025
- WO-A-2004/008961
- WO-A1-99/20984
- US-A- 3 643 652
- US-A- 6 131 571
- US-A1- 2007 062 528

## Description

### BACKGROUND OF THE INVENTION

### Related Application

This application claims priority from U.S. Patent Application No. 61/009,174 which was filed on December 26, 2007.

### Field of the Invention

The present invention relates to medical devices, and more particularly, to endotracheal tubes.

### Description of the Related Art

This section is intended to introduce the reader to various aspects of art that may be related to various aspects of the present invention, which are described and/or claimed below. This discussion is believed to be helpful in providing the reader with background information to facilitate a better understanding of the various aspects of the present invention. Accordingly, it should be understood that these statements are to be read in this light, and not as admissions of prior art.

In the course of treating a patient, a tube or other medical device may be used to control the flow of air, food, fluids, or other substances into and/or out of the patient. One such device is an endotracheal tube (ETT). An ETT is used in anesthesia, intensive care, and emergency medicine for airway management and mechanical ventilation. The BTT is inserted into a patient's trachea (i.e., windpipe) in order to ensure that the airway is not blocked and that air can successfully reach the lungs. The ETT is regarded as the most reliable device currently available for protecting a patient's airway.

An ETT' may be used to measure lung flow. Conventional methods of measuring lung flow generally involve measuring air flow at the "Y" section of an ETT, which is the end of an ETT farthest from the lungs. Using such methods, lung flow is a function of 1) the air flow measured at the "Y" section of the ETT and 2) ETT resistance (ETT_{R}), which is constant for a specific tube. Moreover, these conventional methods generally involve using off-shelf reusable pressure differential flow sensors.

These conventional methods have two significant drawbacks. Firstly, a recent study shows that within approximately two hours of inserting an ETT into a patient, the internal surface of the ETT will be covered by a significant amount of mucosal secretions, and these secretions will unpredictably alter the effective ETTR of the tube. Hence, the accuracy of lung flow measurements will decrease due to the buildup of these secretions, Secondly, the off-shelf pressure differential flow sensors needed to carry out these conventional methods can be expensive.

WO 2004/008961 and WO 01/23035 both describe differential sensors that can be used in an ETT.

### BRIEF DESCRIPTION OF THE DRAWINGS

Advantages of the invention may become apparent upon reading the following detailed description and upon reference to the drawings, in which:
Fig. 1 illustrates an exemplary medical system containing an endotracheal tube, a ventilator, and a monitor in accordance with embodiments of the present invention;
Fig. 2 illustrates an exemplary endotracheal tube with a side cross-sectional view of a pressure differential flow sensor in accordance with embodiments of the present invention; and
Fig. 3 provides a front cross-sectional view of an exemplary pressure differential flow sensor of an endotracheal tube taken along line 3-3 of Fig. 2 in accordance with embodiments of the present invention.

### DETAILED DESCRIPTION OF SPECIFIC EMBODIMENTS

One or more specific embodiments of the present invention will be described below, In an effort to provide a concise description of these embodiments, not all features of an actual implementation are described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

In accordance with the invention, an ETT is provided. A pressure differential flow sensor is built into the ETT at the end closest to the lungs. The airway of the ETT has a fixed diameter throughout, with the exception of a section located within the pressure differential flow sensor, within which the diameter of the airway increases relative to the fixed diameter. The flow sensor functions by sensing the difference in air flow between the airway portion where the diameter is fixed and the airway portion where the diameter is relatively larger.

Using this built-in flow sensor to measure lung flow has two significant advantages over the use of conventional methods. Firstly, since the flow sensor is placed at the end of the ETT closest to the lungs, lung flow will be measured from air flowing directly out of the lungs rather than from air flowing through the length of the ETT. Thus, the buildup of mucosal secretions on the internal surface of the ETT's airway will have significantly less influence upon measurements taken by the flow sensor, allowing for more accurate measurements. Indeed, mucus-induced fluctuations in the effective ETT_{R} value will typically have negligible impact upon measurements taken by the flow sensor. Secondly, since the flow sensor is built into the ETT, using this flow sensor will eliminate the need to purchase an off-shelf reusable pressure differential flow sensor, resulting in reduced expense.

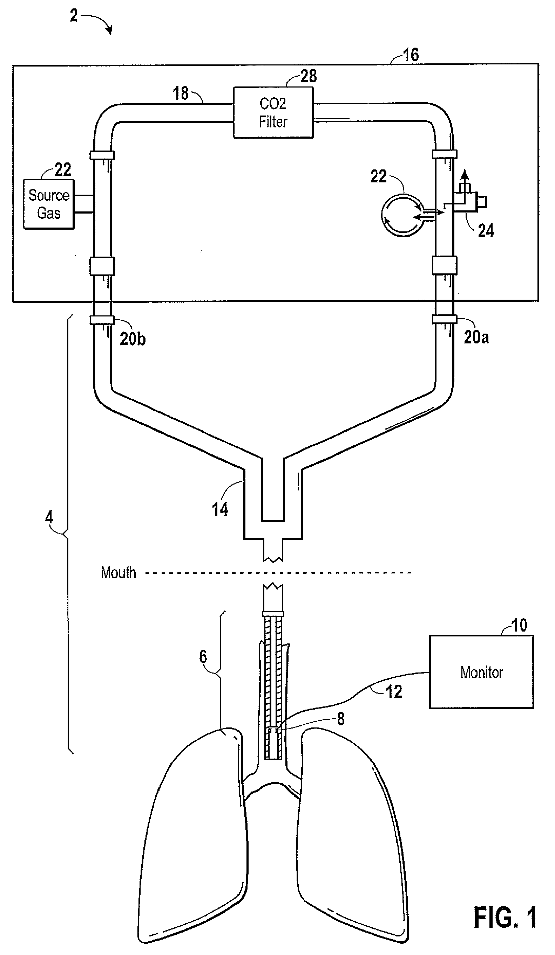

Turning now to the figures, FIG. 1 provides a representative view of a medical system 2 according to an embodiment of the invention. The medical system 2 comprises an endotracheal tube (ETT) 4 according to an embodiment of the invention. The ETT 4 is designed for insertion into the trachea of a patient. The ETT 4 includes a tracheal portion 6, which is defined to comprise the portion of the ETT inserted into the patient's trachea. The tracheal portion 6 is shown in cross-section in FIG. 1 in order to provide further detail regarding the sensor. More specifically, according to the invention, a pressure differential flow sensor 8 is built into the tracheal portion 6 and is positioned at the end of the tracheal portion 6 closest to the patient's lungs. Any type of suitable pressure sensor may be used, including commercially available, e.g., "off-the-shelf", pressure sensors. For example, such sensors may include sensors having a pair of piezo-resistive elements or sensors having a single piezo-resistive element mounted at one location on the tracheal portion 6 and referenced to another location via a connecting gas channel.

The pressure differential flow sensor 8 may be communicatively coupled to a monitor 10. It should be appreciated that the pressure differential flow sensor 8 may be coupled to the monitor 10 via a cable 12. Alternately, the pressure differential flow sensor 8 may be coupled to a transmission device (not shown) that facilitates wireless communication between the pressure differential flow sensor 8 and the monitor 10. The monitor 10 may be configured to determine lung flow based upon information received from the pressure differential flow sensor 8. The monitor 10 may be coupled to a multiparameter patient monitor (not shown).

The ETT 4 may comprise a Y-shaped portion 14 that is configured to couple the ETT 4 to a ventilator 16. The ventilator 16 may comprise a conduit 18 that is configured to connect with both ends of the Y-shaped portion 14. The Y-shaped portion 14 and the conduit 18 facilitate one-way flow of expired gases away from the patient and one-way flow of inspired gases toward the patient. The one-way flow of gases through the medical system 2 may be achieved through the use of in-line one-way valves 20a and 20b. The Y-shaped portion 14 and the conduit 18 may comprise standard medical tubing made from suitable materials such as polyurethane, polyvinyl chloride (PVC), polyethylene teraphthalate (PETP), low-density polyethylene (LDPE), polypropylene, silicone, neoprene, or polyisoprene.

The ventilator 16 may comprise a source gas supply 22 that provides source gas for inspiration by the patient. The source gas provided by source gas supply 22 may comprise respiratory gas mixtures and anesthetic/therapeutic components such as anesthetic agents, nitric oxide, radioactively tagged particles and/or a variety of other gaseous agents. It will be appreciated that the gas stream expired by the patient may also include a combination of respiratory gases and anesthetic/therapeutic gases. Moreover, the ventilator 16 may include a vent 24 and/or a gas reservoir 26 to relieve excess volume or pressure in the medical system 2. Furthermore, the ventilator 16 may comprise a carbon dioxide filter 28 to remove carbon dioxide from the medical system 2 before the fresh source gas is added to the system by the source gas supply 22.

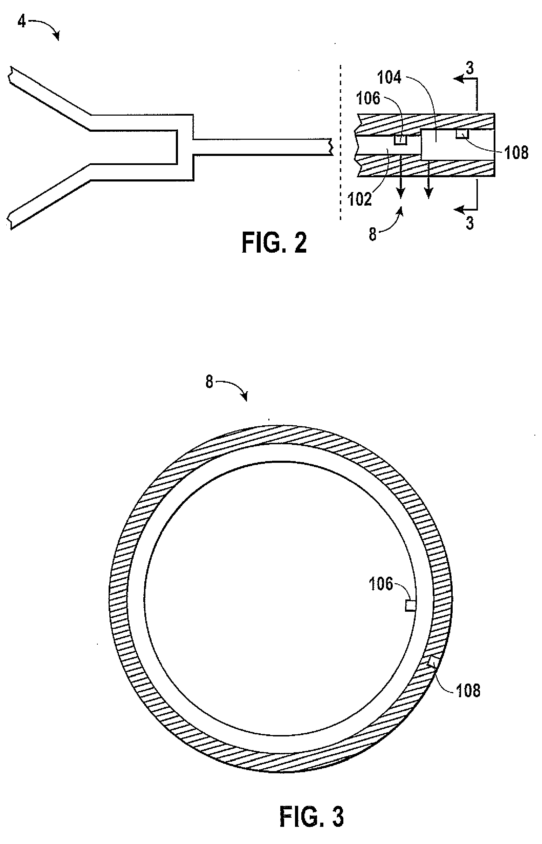

FIG. 2 illustrates the ETT 4 and a side cross-sectional view of the pressure differential flow sensor 8 in further detail in accordance with embodiments of the present invention. Specifically, FIG. 2 illustrates the various components of the pressure differential flow sensor 8. Moreover, FIG. 3 provides a front cross-sectional view of the pressure differential flow sensor 8. The pressure differential flow sensor 8 measures the difference in air flow between an airway portion 102, where the airway diameter is equivalent to the fixed diameter of the ETT 4, and an airway portion 104, where the airway diameter is relatively larger. The measurement is accomplished through the use of two sensors; one sensor 106 is located within the airway portion 102 to measure air flow through the airway portion 102, and a second sensor 108 is located within the airway portion 104 to measure air flow through the airway portion 104. The sensors 106 and 108 may be attached to the surfaces of airway portions 102 and 104 respectively. The measurements recorded by the sensors 106 and 108 may be relayed to the monitor 10 of the medical system 2 to determine lung flow.

In embodiments, the monitor has a graphical display, the monitor is incorporated into the ventilator, and/or the monitor is wirelessly coupled to the pressure differential flow sensor.

While the invention may be susceptible to various modifications and alternative forms, specific embodiments have been shown by way of example in the drawings and have been described in detail herein. However, it should be understood that the invention is not intended to be limited to the particular forms disclosed. The scope of protection is defined by the following appended claims.

## Claims

1. An endotracheal tube (4) configured to transfer gas between a ventilator (16) and a patient's lungs, comprising: a first end configured to connect to the ventilator (16); a second end configured for insertion into the patient's lungs; and a pressure differential flow sensor (8) positioned at the second end, wherein the second end of the tube (4) comprises a first airway portion (102) having a first cross-sectional diameter and a second airway portion (104) having a second cross sectional diameter larger than the first cross sectional diameter, wherein the pressure differential flow sensor (8) is configured to measure a difference in flow between the first airway portion (102) and the second airway portion (104), the endotracheal tube has a fixed diameter and and the first cross-sectional diameter is equivalent to the fixed diameter of the endotracheal tube (4).

2. The endotracheal tube (4) of claim 1, wherein the first end comprises a Y-section.

3. A medical system comprising: a ventilator (16), an endotracheal tube (4) according to claim 1, and a monitor communicatively coupled to the pressure differential flow sensor of the endotracheal tube.

4. The medical system of claim 3, wherein the monitor (10) comprises a monitor with a graphical display.

5. The medical system of claim 3 or 4, wherein the monitor (10) is incorporated into the ventilator (16).

6. The medical system of any of claims 3 to 5, wherein the monitor (10) is wirelessly coupled to the pressure differential flow sensor (8).

7. The medical system of any of claims 3 to 6, wherein the first end comprises a Y-section.

8. A method of manufacturing a medical system, comprising: providing a ventilator (16); providing an endotracheal tube (4) configured to transfer gas between a ventilator (16) and a patient's lungs and comprising a first end configured to connect to the ventilator (16), a second end configured for insertion into the patient's lungs, and a pressure differential flow sensor (8) positioned at the second end, wherein the second end of the tube (4) comprises a first airway portion (102) having a first cross-sectional diameter and a second airway portion (104) having a second cross sectional diameter larger than the first cross sectional diameter, wherein the pressure differential flow sensor (8) is configured to measure a difference in flow between the first airway portion (102) and the second airway portion (104); and providing a monitor (10) communicatively coupled to the pressure differential flow sensor (8), wherein the endotracheal tube has a fixed diameter and the first cross-sectional diameter is equivalent to the fixed diameter of the endotracheal tube (4).

9. The method as set forth in claim 8, wherein providing the monitor (10) comprises providing a monitor with a graphical display.

10. The method as set forth in claim 8 or 9, wherein providing the monitor (10) comprises providing a monitor incorporated into the ventilator (16).

11. The method as set forth in any of claims 8 to 10, wherein providing the monitor (10) comprises providing a monitor wirelessly coupled to the pressure differential flow sensor (8).

12. The method as set forth in any of claims 8 to 11, wherein providing the second end comprises providing a Y-section.

## Patentansprüche

1. Endotrachealtubus (4), dafür gestaltet, Gas zwischen einem Beatmungsgerät (16) und der Lunge eines Patienten zu überführen, und Folgendes umfassend: ein erstes Ende, das dafür gestaltet ist, an das Beatmungsgerät (16) angeschlossen zu sein, ein zweites Ende, das für das Einführen in die Lunge des Patienten gestaltet ist, und einen Druckdifferenz-Strömungssensor (8), der am zweiten Ende positioniert ist, wobei das zweite Ende des Tubus (4) einen ersten Luftwegabschnitt (102) mit einem ersten Querschnittsdurchmesser und einen zweiten Luftwegabschnitt (104) mit einem zweiten Querschnittsdurchmesser, der größer als der erste Querschnittsdurchmesser ist, umfasst, wobei der Druckdifferenz-Strömungssensor (8) dafür konfiguriert ist, eine Differenz der Strömung zwischen dem ersten Luftwegabschnitt (102) und dem zweiten Luftwegabschnitt (104) zu messen, wobei der Endotrachealtubus einen feststehenden Durchmesser aufweist und der erste Querschnittsdurchmesser dem feststehenden Durchmesser des Endotrachealtubus (4) entspricht.

2. Endotrachealtubus (4) nach Anspruch 1, wobei das erste Ende einen Y-Abschnitt umfasst.

3. Medizinisches System, Folgendes umfassend: ein Beatmungsgerät (16), einen Endotrachealtubus (4) nach Anspruch 1 und einen Monitor, der kommunikativ mit dem Druckdifferenz-Strömungssensor des Endotrachealtubus gekoppelt ist.

4. Medizinisches System nach Anspruch 3, wobei der Monitor (10) einen Monitor mit einer graphischen Anzeige umfasst.

5. Medizinisches System nach Anspruch 3 oder 4, wobei der Monitor (10) in das Beatmungsgerät (16) eingebunden ist.

6. Medizinisches System nach einem der Ansprüche 3 bis 5, wobei der Monitor (10) drahtlos mit dem Druckdifferenz-Strömungssensor (8) gekoppelt ist.

7. Medizinisches System nach einem der Ansprüche 3 bis 6, wobei das erste Ende einen Y-Abschnitt umfasst.

8. Verfahren zur Herstellung eines medizinischen Systems, Folgendes umfassend: Bereitstellen eines Beatmungsgeräts (16), Bereitstellen eines Endotrachealtubus (4), der dafür gestaltet ist, Gas zwischen einem Beatmungsgerät (16) und der Lunge eines Patienten zu überführen und ein erstes Ende umfasst, das dafür gestaltet ist, an das Beatmungsgerät (16) angeschlossen zu sein, ein zweites Ende, das für das Einführen in die Lunge des Patienten gestaltet ist, und einen Druckdifferenz-Strömungssensor (8), der am zweiten Ende positioniert ist, wobei das zweite Ende des Tubus (4) einen ersten Luftwegabschnitt (102) mit einem ersten Querschnittsdurchmesser und einen zweiten Luftwegabschnitt (104) mit einem zweiten Querschnittsdurchmesser, der größer als der erste Querschnittsdurchmesser ist, umfasst, wobei der Druckdifferenz-Strömungssensor (8) dafür konfiguriert ist, eine Differenz der Strömung zwischen dem ersten Luftwegabschnitt (102) und dem zweiten Luftwegabschnitt (104) zu messen, und Bereitstellen eines Monitors (10), der kommunikativ mit dem Druckdifferenz-Strömungssensor (8) des Endotrachealtubus gekoppelt ist, wobei der Endotrachealtubus einen feststehenden Durchmesser aufweist und der erste Querschnittsdurchmesser dem feststehenden Durchmesser des Endotrachealtubus (4) entspricht.

9. Verfahren nach Anspruch 8, wobei das Bereitstellen des Monitors (10) das Bereitstellen eines Monitors mit einer grafischen Anzeige umfasst.

10. Verfahren nach Anspruch 8 oder 9, wobei das Bereitstellen des Monitors (10) das Bereitstellen eines Monitors umfasst, der in das Beatmungsgerät (16) eingebunden ist.

11. Verfahren nach einem der Ansprüche 8 bis 10, wobei das Bereitstellen des Monitors (10) das Bereitstellen eines Monitors umfasst, der drahtlos mit der Druckdifferenz-Strömungssensor (8) gekoppelt ist.

12. Verfahren nach einem der Ansprüche 8 bis 11, wobei das Bereitstellen des zweiten Endes das Bereitstellen eines Y-Abschnitts umfasst.

## Revendications

1. Tube endotrachéal (4) configuré pour transférer un gaz entre un ventilateur (16) et les poumons d'un patient, comprenant: une première extrémité configurée pour être connectée au ventilateur (16); une seconde extrémité configurée pour être insérée dans les poumons du patient; et un capteur de débit par pression différentielle (8) positionné au niveau de la seconde extrémité, la seconde extrémité du tube (4) comprenant une première partie de voie respiratoire (102) ayant un premier diamètre de section transversale et une seconde partie de voie respiratoire (104) ayant un second diamètre de section transversale supérieur au premier diamètre de section transversale, le capteur de débit par pression différentielle (8) étant configuré pour mesurer une différence de débit entre la première partie de voie respiratoire (102) et la seconde partie de voie respiratoire (104), le tube endotrachéal ayant un diamètre fixe et le premier diamètre de section transversale étant égal au diamètre fixe du tube endotrachéal (4).

2. Tube endotrachéal (4) selon la revendication 1, la première extrémité comprenant une section en Y.

3. Système médical comprenant un ventilateur (16), un tube endotrachéal (4) selon la revendication 1 et un moniteur couplé de manière communicante au capteur de débit par pression différentielle du tube endotrachéal.

4. Système médical selon la revendication 3, le moniteur (10) comprenant un moniteur à affichage graphique.

5. Système médical selon la revendication 3 ou 4, le moniteur (10) étant incorporé au ventilateur (16).

6. Système médical selon l'une quelconque des revendications 3 à 5, le moniteur (10) étant couplé sans fil au capteur de débit par pression différentielle (8).

7. Système médical selon l'une quelconque des revendications 3 à 6, la première extrémité comprenant une partie en Y.

8. Procédé de fabrication d'un système médical consistant à fournir un ventilateur (16); fournir un tube endotrachéal (4) configuré pour transférer un gaz entre un ventilateur (16) et les poumons d'un patient et comprenant une première extrémité configurée pour être connectée au ventilateur (16), une seconde extrémité configurée pour être insérée dans les poumons du patient et un capteur de débit par pression différentielle (8) positionné au niveau de la seconde extrémité, la seconde extrémité du tube (4) comprenant une première partie de voie respiratoire (102) ayant un premier diamètre de section transversale et une seconde partie de voie respiratoire (104) ayant un second diamètre de section transversale supérieur au premier diamètre de section transversale, le capteur de débit par pression différentielle (8) étant configuré pour mesurer une différence de débit entre la première partie de voie respiratoire (102) et la seconde partie de voie respiratoire (104); et fournir un moniteur (10) couplé de manière communicante au capteur de débit par pression différentielle (8), le tube endotrachéal ayant un diamètre fixe et le premier diamètre de section transversale étant égal au diamètre fixe du tube endotrachéal (4).

9. Procédé selon la revendication 8, la fourniture du moniteur (10) consistant à fournir un moniteur à affichage graphique.

10. Procédé selon la revendication 8 ou 9, la fourniture du moniteur (10) consistant à fournir un moniteur incorporé au ventilateur (16).

11. Procédé selon l'une quelconque des revendications 8 à 10, la fourniture du moniteur (10) consistant à fournir un moniteur couplé sans fil au capteur de débit par pression différentielle (8).

12. Procédé selon l'une quelconque des revendications 8 à 11, la fourniture de la seconde extrémité consistant à fournir une partie en Y.
